(19) Europäisches Patentamt
European Patent Office
Office européen des brevets

(11) Numéro de publication: 0 172 128
B1

(12) # FASCICULE DE BREVET EUROPÉEN

(45) Date de publication du fascicule du brevet: 21.06.89

(51) Int. Cl.⁴: C 07 C 97/07, A 61 K 31/135

(21) Numéro de dépôt: 85450515.4

(22) Date de dépôt: 19.06.85

(54) Application thérapeutique d'aminométhylène-2 indanediones-1,3 substituées sur l'azote.

(30) Priorité: 20.07.84 FR 8411678

(43) Date de publication de la demande: 19.02.86 Bulletin 86/8

(45) Mention de la délivrance du brevet: 21.06.89 Bulletin 89/25

(84) Etats contractants désignés: BE CH DE GB IT LI LU NL

(56) Documents cité:
CHEMICAL ABSTRACTS, vol. 85, no. 8, 23 août 1976, page 35, réf. no. 56706g, Columbus, Ohio, US; E. OZOLA et al.: "Synthesis and pharmacological properties of amino derivatives of 2-alkylamino-methylene-1,3-indandione"
CHEMICAL ABSTRACTS, vol. 93, no. 5, 4 août 1980, page 886, réf. no. 46226b, Columbus, Ohio, US; L. GASJUNA et al.: "N-Substituted 2-aminomethylene-1,3-indandiones"
CHEMICAL ABSTRACTS, vol. 77, no. 13, 25 septembre 1972, page 425, réf. no. 88122q, Columbus, Ohio, US; E. OZOLA et al.: "Aminomethylene deri andione"
CHEMICAL ABSTRACTS, vol. 78, no. 21, 28 mai 1973, page 351, réf. no. 135921g, Columbus, Ohio, US; E.M. BELEVICH et a.: "Oxyalkylation reactions in cyclic beta-diketones. IV. 2-Aminomethylene derivatives of 1,3-indandione"

(73) Titulaire: SOCIETE CORTIAL S.A.
7 rue de l'Armorique
F-75015 Paris (FR)

(72) Inventeur: Le Baut, Guillaume, Prof.
5 rue de la Baugerie
F-44230 St-Sébastian-sur-Loire (FR)
Inventeur: Sparfel, Louis, Prof.
8 rue François Marchais
F-44400 Rèze-lès-Nantes (FR)
Inventeur: Creuzet, Marie-Hélène
Jardin de Gambetta T 3
F-33000 Bordeaux (FR)
Inventeur: Feniou, Claude
5 rue Général Guillaumat
F-33600 Pessac (FR)
Inventeur: Pontagnier, Henri
21 rue Edouard-Vaillant
F-33600 Pessac (FR)
Inventeur: Prat, Gisèle
Hameau de Noailles Villa 18
F-33400 Talence (FR)

(74) Mandataire: Tajan, Marie-Thérèse
LABORATOIRES SARGET Avenue du Président JF Kennedy
F-33701 Mérignac (FR)

EP 0 172 128 B1

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe dipposition (Art. 99(1) Convention sur le brevet européen).

LIBERGRAF, STOCKHOLM 1990

**Description**

La présente invention concerne l'application thérapeutique d'aminométhylène-2 indanediones-1,3 substituées sur l'azote.

Les produits faisant l'objet de la présente invention ont pour formule générale

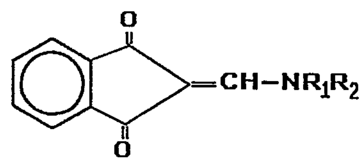

(I)

dans laquelle

$R_1$ représente un atome d'hydrogène ou un groupement alkyl inférieur tel que méthyl ou éthyl,

$R_2$ représente un groupement cyclopropylméthyl, turyl-2 méthyl, benzyl, phényl, ou alkyl inférieur de $C_1$ à $C_4$ linéaire ou ramifié tel que méthyl, éthyl, propyl, isopropyl, butyl, isobutyl, tertiobutyl,

le groupement $NR_1R_2$ pouvant également représenter un hétérocycle azoté $N(CH_2)$ avec n pouvant prendre l'une quelconque des valeurs comprises entre 4 et 6.

Les produits de formule générale (I) ont déjà été décrits dans la littérature; on peut par exemple citer les travaux de Gasjuna L. et coll, Latv. P.S.R. Zinat. Akad. Vest. Kim. Ser. 1980, 1, 98 - 101, où une forte action sur le système nerveux-central leur est attribuée; ainsi que les travaux d'Ozola E. et coll., Kim.-Farm. Zh. 1976, 10 (3), 45 - 50, où un effet tranquillisant leur est attribué.

Nous avons maintenant trouvé que les produits selon la formule (I) présentent des propriétés pharmacologiques notamment dans le traitement de l'hypertension artérielle.

Les produits de la présente invention sont préparés de façon générale par une réaction mettant en jeu la formyl-2 indanedione-1,3 et l'amine de formule générale

$HNR_1R_2$

dans laquelle

$R_1$ représente un atome d'hydrogène ou un groupement alkyl inférieur tel que méthyl ou éthyl,

$R_2$ représente un groupement cyclopropylméthyl, furyl-2 méthyl, benzyl, phényl, ou alkyl inférieur de $C_1$ à $C_4$ linéaire ou ramifié tel que méthyl, éthyl, propyl, isopropyl, butyl, isobutyl, tertiobutyl

et dans laquelle le groupement $NR_1R_2$ peut également représenter un hétérocycle azoté $N(CH_2)$ avec n pouvant prendre l'une quelconque des valeurs comprises entre 4 et 6. Une variante consiste à faire réagir la formyl-2 indanedione-1,3 et le chlorhydrate de l'amine $HNR_1R_2$ définie plus haut, en présence d'un accepteur d'hydracide tel que le carbonate de sodium.

Les exemples exposés ci-après vont permettre de mieux comprendre la présente invention sans toutefois en limiter la portée.

**Exemple I**

Méthylaminométhylène-2 indanedione-1,3; produit de formule (I) dans laquelle $R_1$ = H et $R_2$ = $CH_3$; nom de code COR3726.

**Préparation**

Une solution de 35 ml d'orthoformiate d'éthyle et 70 ml d'anhydride acétique est ajoutée à 25 g d'indanedione-1,3. Le mélange est chauffé à 80°C pendant une heure. La solution est filtrée et refroidie immédiatement dans un bain de glace. 130 ml d'eau préalablement bouillie sont ajoutés sous agitation en veillant à ce que la température se maintienne à 10°C. On laisse cristalliser au froid pendant 5 h. On filtre. Le rendement est de 67 %. Le précipité est versé dans 400 ml d'éthanol bouillant; le mélange est porté au reflux pendant 5 mn puis filtré dans une fiole préalablement chauffée. La solution contient 20 g de formyl-2 indanedione-1,3 et doit être utilisée rapidement.

A une solution de méthylamine en excès dans 2 ml d'acide acétique et 50 ml d'éthanol, on ajoute une solution de 1,2 g de formyl-2 indanedione-1,3 dans 50 ml d'éthanol. Le précipité jaune est filtré et lavé à l'éthanol froid. Rendement 86 %.

**Propriétés physicochimiques**

Point de fusion: 254°C (éthanol), se sublime.

Spectre infrarouge: bandes ν(CO) à 1705 et 1650 $cm^{-1}$.

Spectre de RMN dans le DMSO D6: 9,5 ppm, singulet, 1 proton, NH; 8 ppm, singulet, 1 proton, CH; 7,73 ppm, multiplet, 4 protons, H aromatiques phtaloyle; 3,3 ppm, singulet, 3 protons, $CH_3$.

**Exemple II**

Ethylaminométhylène-2 indanedione-1,3; produit de formule (I) dans laquelle $R_1$ = H et $R_2$ = $C_2H_5$; nom de code COR3755.

**Préparation**

4 ml de solution aqueuse d'éthylamine à 70 % sont ajoutés à une solution de 2,4 g de formyl-2 indanedione-1,3 dans 40 ml d'éthanol. Le mélange est chauffé légèrement au bain-marie pendant 15 mn. Après refroidissement, le produit formé précipite. Il est recristallisé dans l'éthanol. Rendement 86,7 %.

**Propriétés physicochimiques**

Point de fusion: 170°C (éthanol).

Spectre de RMN dans $CDCl_3$: 9,33 ppm, singulet, 1 proton, NH; 8 ppm, singulet, 1 proton, CH; 7,75 ppm, multiplet, 4 protons, H aromatiques phtaloyle; 3,55 ppm, multiplet, 2 protons, $CH_2$; 1,38 ppm, triplet, 3 protons, $CH_3$.

**Exemple III**

Anilinométhylène-2 indanedione-1,3; produit de formule (I) dans laquelle $R_1$ = H et $R_2$ = $C_6H_5$; nom de code COR3756.

**Préparation**

3 g d'aniline sont ajoutés à une solution de 2,4 g de formyl-2 indanedione-1,3 dans 40 ml d'éthanol. Le mélange est chauffé légèrement au bain-marie pendant 20 mn. Après refroidissement, le produit formé précipite. Il est recristallisé dans l'éthanol. Rendement 67,1 %.

**Propriétés physicochimiques**

Point de fusion: 197°C (éthanol).

Spectre de RMN dans $CDCl_3$: 11 ppm, multiplet, 1 proton, NH; 8,38 ppm, singulet, 1 proton, H en 4 sur l'aniline; 8,13 ppm, singulet, 1 proton, CH; 7,77 ppm, multiplet, 4 protons, H aromatiques phtaloyle; 7,38 ppm, multiplet, 4 protons, H en 2, 3, 5, 6 sur l'aniline.

**Exemple IV**

Isobutylaminométhylène-2 indanedione-1,3; produit de formule (I) dans laquelle $R_1$ = H et $R_2$ = $CH_2CH(CH_3)_2$; nom de code COR3754.

**Préparation**

3 g d'isobutylamine sont ajoutés à une solution de 2 g de formyl-2 indanedione-1,3 dans 40 ml d'éthanol. Le mélange est chauffé légèrement au bain-marie pendant 10 mm. L'éthanol est éliminé par évaporation, et le produit attendu est recristallisé dans l'éthanol. Rendement 56 %.

**Propriétés physicochimiques**

Point de fusion: 149°C (éthanol).

Spectre de RMN dans $CDCl_3$: 9,42 ppm, singulet, 1 proton, NH; 7,93 ppm, singulet, 1 proton, = CH; 7,77 ppm, multiplet, 4 protons, H aromatiques phtaloyle; 3,3 ppm, d doublet, 2 protons, $CH_2$; 1,98 ppm, multiplet, 1 proton, CH; 1,025 ppm, doublet, 6 protons, $CH_3$.

**Exemple V**

Isopropylaminométhylène-2 indanedione-1,3; produit de formule (I) dans laquelle $R_1$ = H et $R_2$ = $CH(CH_3)_2$; nom de code COR3762.

**Préparation**

2 ml d'isopropylamine sont ajoutés à une solution de 2,7 g de formyl-2 eindanedione-1,3 dans 60 ml d'éthanol. Le mélange est chauffé au bain-marie pendant 30 mn. L'éthanol est éliminé par évaporation et le produit attendu est recristallisé dans un mélange éther isopropylique/éthanol (9/1) puis dans l'éthanol. Rendement 60 %.

**Propriétés physicochimiques**

Point de fusion: 122°C (éthanol).

Spectre de RMN dans $CDCl_3$: 9,3 ppm, singulet, 1 proton, NH; 8,05 ppm, singulet, 1 proton, = CH; 7,83 ppm, multiplet, 4 protons, H aromatiques phtaloyle; 3,77 ppm, multiplet, 1 proton, CH; 1,41 ppm, doublet, 6 protons, $CH_3$.

**Exemple VI**

Cyclopropylméthylaminométhylène-2 indanedione-1,3; produit de formule (I) dans laquelle $R_1$ = H et $R_2$ =

$CH_2CH$ $CH_2$
\ /
$CH_2$

nom de code COR3757.

**Préparation**

3 g de chlorhydrate de cyclopropylméthylamine et 2,9 g de carbonate de sodium sont ajoutés à une solution de 2,4 g de formyl-2 indanedione-1,3 dans 40 ml d'éthanol. Le mélange est chauffé au bain-marie pendant 30 mn, puis filtré à chaud. Après refroidissement, le produit formé précipite; il est recristallisé dans l'éthanol. Rendement 43 %.

**Propriétés physicochimiques**

Point de fusion: 157°C (éthanol).

Spectre de RMN dans $CDCl_3$: 9,47 ppm, singulet, 1 proton, NH; 8 ppm, singulet, 1 proton, = CH; 7,78 ppm, multiplet, 4 protons, H aromatiques phtaloyle; 3,37 ppm, d doublet, 2 protons. $CH_2$; 1,1 ppm, multiplet, 1 proton, CH; 0,7 ppm, multiplet, 2 protons, $CH_2$ cyclique; 0,38 ppm, multiplet, 2 protons, $CH_2$ cyclique.

**Exemple VII**

(Furyl-2 méthylaminométhylène)-2 indanedione-1,3; produit de formule (I)
dans laquelle $R_1$ = H et $R_2$ =

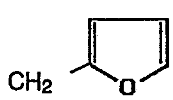

nom de code COR3758

**Préparation**

2,7 g de furyl-2 méthylamine sont ajoutés à une solution de 2,4 g de formyl-2 indanedione-1,3 dans 40 ml d'éthanol. Le mélange est chauffé au bain-marie pendant 20 mn, puis filtré après refroidissement. Le précipité est recristallisé dans le dioxanne. Rendement 40,4 %.

**Propriétés physicochimiques**

Point de fusion: 216°C (dioxanne).

Spectre de RMN dans $CDCl_3$: 9,38 ppm, singulet, 1 proton, NH; 7,97 ppm, singulet, 1 proton, CH; 7,77 ppm, multiplet, 4 protons, H aromatiques phtaloyle; 7,5 ppm, multiplet, 1 proton, H en 5 sur le noyau furyle; 6,45 ppm, multiplet, 2 protons, H en 3 et 4 sur le noyau furyle; 4,67 ppm, singulet, 1 proton, $CH_2$; 4,53 ppm, singulet, 1 proton, $CH_2$.

**Exemple VIII**

Pipéridinométhylène-2 indanedione-1,3; produit de formule (I) dans laquelle $NR_1R_2$ = $N(CH_2)_5$ ; nom de code COR3759.

**Préparation**

2,5 g de pipéridine sont ajoutés à une solution de 2,4 g de formyl-2 indanedione-1,3 dans 40 ml d'éthanol. Le mélange est chauffé au bain-marie pendant 20 mn, puis filtré après refroidissement. Le précipité est recristallisé dans l'éthanol. Rendement 44 %.

**Propriétés physicochimiques**

Point de fusion: 167°C (éthanol).

Spectre de RMN dans $CDCl_3$ : 7,77 ppm, multiplet, 4 protons, H aromatiques phtaloyle; 7,6 ppm, singulet, 1 proton, CH; 4,53 ppm, multiplet, 2 protons, $CH_2$ en 2 ou 6 sur le noyau pipéridine; 3,67 ppm, multiplet, 2 protons, $CH_2$ en 2 ou 6 sur le noyau pipéridine; 1,83 ppm, multiplet, 6 protons, $CH_2$ en 3, 4 et 5 sur le noyau pipéridine.

**Exemple IX**

Diméthylaminométhylène-2 indanedione-1,3; produit de formule (I) dans laquelle $R_1$ = $R_2$ = $CH_3$; nom de code COR3761.

**Préparation**

On ajoute 5 ml d'une solution à 25 % de diméthylamine dans l'éthanol à une solution de 2,2 g de formyl-2 indanedione-1,3 dans 60 ml d'éthanol. Le mélange est chauffé à environ 40°C pendant 4 heures. Le solvant est éliminé par évaporation; le précipité est isolé, puis recristallisé dans un mélange éther isopropylique - éthanol (8/2). Rendement 58 %.

**Propriétés physicochimiques**

Point de fusion: 138°C (éthanol).

Spectre de RMN dans $CDCl_3$: 2,8 ppm, multiplet, 4 protons, H aromatiques phtaloyle; 7,6 ppm, singulet, 1 proton, = CH; 3,83 ppm, singulet, 3 protons, $CH_3$; 3,42 ppm, singulet, 3 protons, $CH_3$.

**Exemple X**

Perhydroazépinyl-1 méthylène-2 indanedione-1,3; produit de formule (I) dans laquelle $NR_1R_2$ = $N(CH_2)_6$; nom de code COR3790.

**Préparation**

3 ml d'hexaméthylèneimine sont ajoutés à une solution de 1,6 g de formyl-2 indanedione-1,3 dans 40 ml d'éthanol. Le mélange est chauffé au bain-marie pendant 60 mm. L'éthanol est évaporé partiellement; le résidu est placé au froid pendant 12 heures. Le précipité est filtré puis purifié par passage sur colonne de silice en éluant par de l'éther éthylique. Rendement 43 %.

**Propriétés physicochimiques**

Point de fusion: 125°C (éther éthylique).

Spectre IR: bandes νCO à 1700 et 1650 $cm^{-1}$.

Spectre de RMN dans $CDCl_3$: 7,78 ppm, multiplet, 4 protons, H aromatiques phtaloyle; 7,63 ppm, singulet, 1 proton, CH; 4,50 ppm, multiplet, 2 protons, $CH_2$ en 2 ou 7 sur le noyau perhydroazépinyle; 3,67 ppm, multiplet, 2 protons, $CH_2$ en 2 ou 7 sur le noyau perhydroazépinyle; 1,73 ppm, multiplet, 8 protons, $CH_2$ en 3, 4, 5 et 6 sur le noyau perhydroazépinyle.

Les résultats d'études toxicopharmacologiques réalisées sur les produits selon la présente invention sont exposés ci-après.

**Toxicite**

Administrés à la souris par voie orale à la dose de 300 mg/kg ou par voie intrapéritonéale à la dose de 200 mg/kg, les COR3726, COR3754, COR3755, COR3756, COR3757, COR3758, COR3759, COR3761, COR3762, COR3790 n'induisent aucune mortalité. La DL50 du COR3761 administré à la souris par voie orale en solution dans le Tween 20 % est égale à 520 (457-592) mg/kg.

**Pharmacologie**

L'activité diurétique a été déterminée sur plusieurs modèles expérimentaux. Chez le rat soumis à une surcharge hydrique, administrés par voie orale à la dose de 20 mg/kg, le COR3726 et le COR3761 multiplient l'excrétion urinaire du sodium par respectivement un facteur 4,4 et 3,5. La spironolactone administrée dans les mêmes conditions et à la même dose multiplie l'excrétion sodique par 3,6. Administré par voie orale au rat soumis à une surcharge saline, le COR3761 augmente l'excrétion urinaire volumétrique mesurée à la sixième heure de 10 % à la dose de 10 mg/kg, 42,5 % à la dose de 20 mg/kg et 68,4% à la dose de 40 mg/kg. Le COR3761 a été administré au rat soumis à une surcharge hydrique et l'on a mesuré, sur une période de 6 heures, l'excrétion urinaire volumétrique, le sodium et le potassium excrétés. Ces paramètres varient de respectivement -13 %, +73 %, +77 % après administration de 10 mg/kg, +9 %, +241 %, +114 % après administration de 20 mg/kg et de -4 %, +532 %, +138 % après administration de 40 mg/kg; le rapport Na/K qui était de 0,4 chez les animaux témoins est de 0,47 à 10, 0,62 à 20 et 1,48 à 40 mg/kg. Au cours d'une autre expérimentation, l'excrétion urinaire volumétrique, le sodium et le potassium excrétés ont varié de respectivement +1 %, +100 %, +54 % à 20 mg/kg, +10 %, +313 %, +69 % à 40 mg/kg, et +16 %, +356 % et 89 % à 60 mg/kg; le rapport Na/K est passé de 0,43 chez les animaux témoins à 0,53 à 20 mg/kg, 1,02 à 40 mg/kg et 1,12 à 60 mg/kg.

Compte tenu de l'activité diurétique jointe à une faible toxicité, les produits faisant l'objet de la présente invention pourront être utilisés en thérapeutique humaine ou vétérinaire dans le traitement de l'hypertension artérielle.

Associés aux excipients habituels, ils seront administrés, selon l'indication, par exemple par voie orale sous forme de dragées, comprimés, sirops, ampoules, par voie rectale sous forme de suppositoires, par voie intramusculaire ou intraveineuse. Les doses administrées varieront selon l'indication et le sujet de 1 à 100 mg/j en 1 à 6 prises pour la voie orale, de 1 à 100 mg/j en 1 ou 2 prises pour la voie rectale, de 0,5 à 50 mg par injection pour les voies parentérales.

**Revendication**

1. Utilisation d'au moins un produit de formule générale:

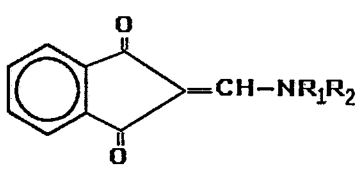

dans laquelle

$R_1$ représente un atome d'hydrogène ou un groupement alkyl inférieur tel que méthyl ou éthyl,

$R_2$ représente un groupement cyclopropylméthyl, furyl-2 méthyl, benzyl, phényl, ou alkyl inférieur de $C_1$ à $C_4$ linéaire ou ramifié tel que méthyl, éthyl, propyl, isopropyl, butyl, isobutyl, tertiobutyl, le groupement $NR_1R_2$ pouvant également représenter un hétérocycle azoté

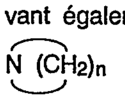

avec n pouvant prendre l'une quelconque des valeurs comprises entre 4 et 6, comme principe actif pour la préparation d'un médicament utile pour le traitement de l'hypertension artérielle.

**Patentanspruch**

1. Verwendung mindestens einer Verbindung der allgemeinen Formel:

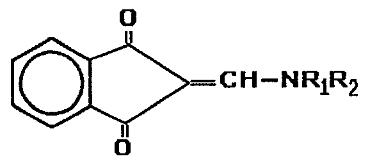

in der

$R_1$ ein Wasserstoffatom oder eine niedere Alkylgruppe wie Methyl oder Ethyl und

$R_2$ eine Cyclopropylmethyl-, Furyl-2-methyl-, Benzyl-, Phenyl- oder eine niedere, lineare oder verzweigte $C_1$- bis $C_4$-Alkylgruppe wie Methyl, Ethyl, Propyl, Isopropyl, Butyl, Isobutyl oder tert. Butyl bedeuten, wobei die Gruppe $NR_1R_2$ auch ein stickstoffhaltiger Heterocyclus der Formel

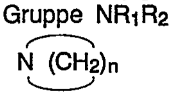

sein kann, in der n eine der Zahlen von 4 bis 6 ist, als Wirkstoff für die Herstellung eines Arzneimittels zur Behandlung des arteriellen Hochdrucks.

**Claim**

1. Use of at least one product of the general formula:

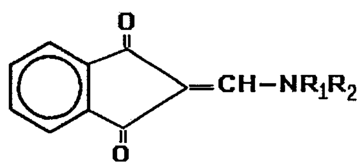

in which

$R_1$ represents a hydrogen atom or a lower alkyl group, such as a methyl or ethyl group,

$R_2$ represents a cyclopropyl methyl, 2-furyl methyl, benzyl, phenyl group or a linear or ramified $C_1$-$C_4$ lower alkyl group, such as methyl, ethyl, propyl, isopropyl, butyl, isobutyl, t-butyl; the group $NR_1R_2$ may also represent a nitrogen heterocycle

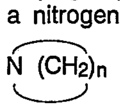

wherein n may have any of the values between 4 and 6, as active principle for preparation of a pharmaceutical product useful in the treatment of the arterial hypertension.